(19) Europäisches Patentamt
European Patent Office
Office européen des brevets

(11) Publication number: **0 089 857**
**B1**

(12) **EUROPEAN PATENT SPECIFICATION**

(45) Date of publication of patent specification: **22.01.86**

(51) Int. Cl.[4]: **C 07 C 69/90**, C 07 C 67/14, A 61 K 31/00// C07C69/24, C07C67/28

(21) Application number: **83301672.8**

(22) Date of filing: **24.03.83**

(54) **Pharmaceutical compounds and process for the manufacture of same.**

(30) Priority: **24.03.82 AU 3284/82**

(43) Date of publication of application: **28.09.83 Bulletin 83/39**

(45) *Publication of the grant of the patent:* **22.01.86 Bulletin 86/04**

(84) Designated contracting states: **AT BE CH DE FR GB IT LI NL SE**

(56) References cited:
**GB-A- 456 269**
**US-A- 2 070 240**
**The file contains technical information submitted after the application was filed and not included in this specification**

(73) Proprietor: **R.P. SCHERER CORPORATION**
**2075 West Big Beaver Road**
**Troy Michigan 48099 (US)**

(72) Inventor: **Bateman, Neil E.**
**23 Edro Avenue East Brighton**
**Melbourne Victoria (AU)**
Inventor: **Woods, Ross A.**
**Flat 3 10 Cook Street**
**West Brunswick Victoria (AU)**

(74) Representative: **Allam, Peter Clerk et al**
**LLOYD WISE, TREGEAR & CO. Norman House**
**105-109 Strand**
**London WC2R 0AE (GB)**

**EP 0 089 857 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

The present invention relates to new pharmaceutical compounds and to a process for the preparation thereof. In particular, the present invention relates to analgesic and anti-inflammatory compounds.

Acetyl salicylic acid, or aspirin as it is more commonly known, is amongst the most widely used of proprietary medicines. Aspirin can be used in the treatment of numerous ailments and is indicated to have analgesic, anti-inflammatory, antipyretic and antirheumatic activity. However, side effects of the drug may limit its application. For example, acetyl salicylic acid may cause gastric irritation and is contra-indicated where such irritation must necessarily be avoided.

The object of the present invention is to provide pharmaceutical substances and compositions which exhibit some or all of the advantageous properties of aspirin but in which the contra-indications are minimized.

According to the present invention it has been discovered that certain ester derivatives of acetyl salicylic acid provide useful pharmaceutical activity with minimal contra-indications.

Specifically, the invention provides an acetyl salicylic derivative of the formula:

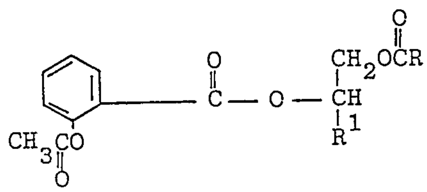

(I)

wherein R and $R^1$, which may be the same or different, represent hydrocarbon radicals. The present invention further provides pharmaceutically acceptable acid addition salts of the compounds of formula I.

In a preferred form R may represent an alkyl group. The alkyl group may include for example from 1 to 15 carbon atoms. The alkyl group may be a straight chain alkyl group. In particularly preferred form R represents a straight chained alkyl group of from 7 to 11 carbon atoms. In a preferred form of the invention $R^1$ represents a lower alkyl group, for example, a methyl, ethyl or propyl group. A methyl group has been found most satisfactory.

Thus, a preferred group of compounds of the present invention are of the formula II:

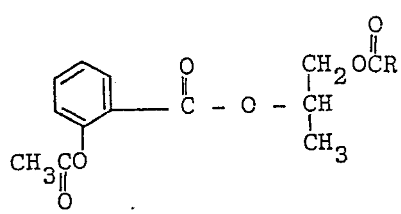

(II)

where R is a straight chain alkyl radical having from 1 to 15 carbon atoms and preferably from 7 to 11 carbon atoms.

The compounds of the present invention have analgesic and anti-inflammatory activity similar to those of acetyl salicylic acid. It has been found that the following tests provided a valuable guide to the activity of the compounds of the present invention. These tests are the animal model experiments described by L.B. Witkin et al., J. Pharmacol. Exptl. Therap., 133, 400 (1961) and C.A. Winter et al., Proc Soc, Exp. Biol. Med., 111, 544 (1962). The use of compounds of the formula I is particularly advantageous since gastric irritation associated with acetyl salicylic acid therapy is avoided or at least minimized.

The salicylic derivatives of the present invention may be manufactured by any chemical process known to be useful for the manufacture of chemically analagous compounds. A preferred process for the manufacture of a salicylate derivative of the present invention comprises the reaction of an acetyl salicylic acid derivative with a monoglycol ester of the general formula III:

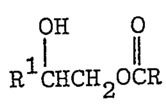

(III)

wherein R and $R^1$ have the meanings given above. An acid halide derivative of acetyl salicylic acid, that is an, O-acethyl salicyloyl halide such as O-acetyl salicyloyl chloride may be used. The reaction may be undertaken in an inert organic solvent, for example, carbon tetrachloride, chloroform, dichloromethane, acetonitrile, ether, tetrahydrofuran or dioxane or the like.

Compounds of the formula III may be prepared via an acylation reaction of a hydroxyketone of the formula IV:

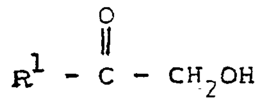 (IV)

wherein $R^1$ has the meaning given above ; with a carboxylic acid of the formula V :

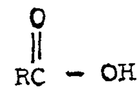 (V)

wherein R has the meaning given above ; to yield compounds of the formula VI :

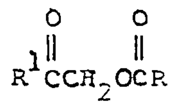 (VI)

A particularly preferred hydroxyketone is the monohydroxy acetone. This yields a monoglycol ester of the formula VII :

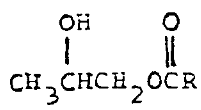 (VII)

This subsequently provides a salicylic acid derivative of the formula

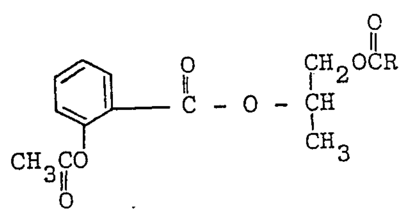 (II)

These reactions may be achieved by standard procedures. For example, the acid V may be activated by conversion to an acid halide e. g. the acid chloride prior to coupling with the hydroxyketone. Alternatively the hydroxyketone may be reacted directly with the acid V in the presence of a condensing agent. The condensing agent may be a carbodiimide such as N,N-dicyclohexylcarbodiimide. For a review of the methods reference may be made to J. March, « Advanced Organic Chemistry : Reactions, Mechanisms and Structure », McGraw-Hill, New York 1968, p. 319-324.

The process is completed by reduction of the intermediates of the formula VI to compounds of formula III. The reduction may be achieved by use of a reducing agent. The reducing agent may be sodium borohydride for example.

Particularly preferred compounds of the present invention exhibiting analgesic and anti-inflammatory activity but with reduced gastric irritation include 2-(1-Decanoyloxypropyl) O-Acetylsalicylate.

A suitable acid-addition salt of a salicylate derivative of the invention is, for example, a salt derived from an inorganic acid, for example, a hydrochloride, hydrobromide, phosphate or sulphate, or a salt derived from an organic acid, for example an oxalate, lactate, succinate, tartrate, acetate, salicylate, citrate, benzoate, B-naphthoate, or adipate.

The salicylate derivatives of the present invention may be administered to animals including man, in the form of a pharmaceutical composition comprising as active ingredient at least one salicylate derivative of the present invention, or an acid-addition salt thereof, in association with a pharmaceutically acceptable diluent or carrier therefor. The composition may further comprise a physiologically acceptable excipient, binder, preservative, stabilizer, flavoring or other compounding ingredient. The composition may be prepared in a conventional unit dosage form as called for by accepted pharmaceutical practice. The composition may be included in soft gelatin capsules, two piece hard gelatin shell capsules, tablets, elixirs, suspensions, emulsions or in injectible solutions or suspensions or oily solutions or suspensions.

The amount of active substances included is selected so as to provide an individual unit dosage, preferably from about 30 milligrams to 1 500 milligrams of the active ingredient. Other therapeutically valuable substances may also be included. Caffeine, phenacetin, paracetamol may be used in addition to the active ingredient of the present invention.

The following examples are illustrative of the invention, or of intermediates which may lead to the

invention, and represent preferred embodiments but are not to be construed as being limitations thereon. All temperatures are in degrees Celsius.

Example 1 : 1-Decanoyloxypropan-2-one

Monohydroxyacetone was distilled prior to use. Chloroform was distilled from phosphorous pentoxide prior to use. Decanoyl chloride (65 $cm^3$, 0.314 6 mole) was added dropwise to stirred solution of monohydroxyacetone (21.8 $cm^3$, 0.3146 mole) and anhydrous pyridine (25.4 $cm^3$, 0.3146 mole) in anhydrous chloroform (500 $cm^3$). During the addition the solution was maintained in an ice bath. The mixture was then stirred at room temperature overnight.

The solution was washed with water (4 × 200 $cm^3$) and saturated sodium chloride solution (200 $cm^3$). The organic phase was dried over sodium sulphate and evaporated *in vacuo*. The resulting oil was distilled to yield 1-decanoyloxypropan-2-one as a mobile liquid, bp 154-60° (1 mm) (52.4 g, 0.230 mole, 73 %), ir (neat) 2 860, 1 700, 1 400, 1 340, 1 150, 1 110 and 1 050 $cm^{-1}$ ; pmr ($CDCl_3$), 4.6 (s, 2 H), 2.4 (t, 2 H, 2.1 (s, 3 H), 1.8-1.0 (bm, 14 H) and 0.9 (t, 3 H) ppm.

Example 2

1-Decanoyloxypropan-2-one (44.3 g, 0.194 mole) was dissolved in a solution of tetrahydrofuran (1 100 $cm^3$) and benzene (200 $cm^3$). After cooling to 5°, ice water (80 $cm^3$) was added. Sodium borohydride (11 g, 0.291 mole) was added to the stirred solution in small proportions to maintain the temperature at 5°. After addition the reaction mixture was stirred at 5° for 45 minutes and glacial acetic acid (14 $cm^3$) was added dropwise. Stirring at 5° was continued for a further 30 minutes. Diethyl ether and chloroform (200 $cm^3$ each) were added and the mixture washed with water (2 × 200 $cm^3$), a 1 % sodium bicarbonate solution (200 $cm^3$) and brine (200 $cm^3$). The organic phase was dried over sodium sulphate and evaporated to yield 1-decanoyloxypropan-2-ol as a colorless liquid (44.0 g, 0.191 mole, 98 %), ir (neat) 3 600-3 100, 2 920, 2 860, 1 730, 1 460, 1 375, 1 240, 1 175, 1 110, 1 055 $cm^{-1}$ ; pmr ($CDCl_3$) 4.0 (m, 2 H), 3.6 (m, 2 H), ($D_2$) (m, 1 Hm, 2.3 (t, 2 H), 2.0-1.0 (bm, 17 H) and 0.85 (t, 3 H) ppm.

Example 3 : 2-(1-Decanoyloxypropyl) O-acetylsalicylate

A solution of O-acetylsalicyloyl chloride (12.5 g, 0.0629 mole) in dry carbontetrachloride (25 $cm^3$) was added slowly to a stirred solution of 1-decanoyloxypropan-2-ol (4.85 g, 0.0211 mole) and dry pyridine (5.1 $cm^3$, 0.0630 mole) in dry carbontetrachloride (150 $cm^3$) at 5°. The solution was then stirred at 25° for two hours and poured over ice (100 g). The mixture was stirred for two hours and the organic layer separated, washed with water, (2 × 150 $cm^3$), 1 % hydrochloric acid, water, 1 % sodium bicarbonate, water and brine (2 × 100 $cm^3$ each), dried over sodium sulphate and evaporated *in vacuo*. The product was chromatographed on silica gel (500 g) in petroleum ether (40-60°) : ether (80 : 20) and treated whith charcoal in acetone to yield 2-(1-decanoyloxypropyl) O-acetylsalicyate as a colorless liquid (3.4 g. 0.00866 mole, 41 %) ; ir (neat) 2 920, 2 850, 1 770, 1 725, 1 605, 1 480, 1 450, 1 365, 1 290, 1 250, 1 190, 1 160, 1 115, 1 075, and 915 $cm^{-1}$ ; pmr ($CDCl_3$) 7.9 (dd, 1 H), 7.6-6.9 (m, 3 H), 5.3 (m, 1 H), 4.2 (m, 2 H), 2.3 (2.3 H), 2.3 (m, 2 H), 1.9-1.0 (bm, 17 H) and 0.9 (t, 3 H) ppm. Anal. calcd, for $C_{23}H_{32}O_6$ : C, 67.32 ; H 8.22 %. Found : C, 6725 ; H, 8.07 %.

Example 4 : 1-Dodecanoyloxypropan-2-ol

By replacing decanoyl chloride in Example 1 with dodecanoyl chloride, 1-dodecanoyloxypropan-2-one and subsequently 1-dodecanoyloxypropan-2-ol, as in Example 2, can be obtained.

Example 5 : 2-(1-Dodecanoyloxypropyl) O-Acetylsalicylate)

By replacing 1-decanoyloxypropan-2-ol with 1-dodecanoyloxypropan-2-ol in Example 3, 2(1-dodecanoyloxypropyl) O-acetylsalicylate can be obtained.

**Claims** (for the Contracting States : BE, CH, DE, FR, GB, IT, LI, NL, SE)

1. A compound having the formula :

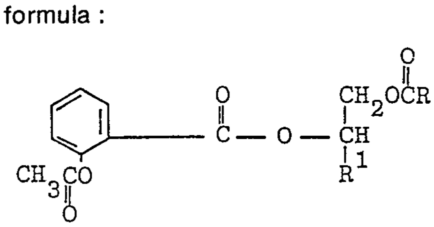

(I)

wherein R and $R^1$ are the same or different hydrocarbon radicals ; or a pharmaceutically acceptable acid addition salt thereof.

2. A compound of Claim 1, wherein R is an alkyl group having 1 to 15 carbon atoms.
3. A compound of Claim 2, wherein said alkyl group is a straight chain alkyl group.
4. A compound of Claim 3, wherein said straight chain alkyl group has 7 to 11 carbon atoms.
5. A compound of any preceding claim, wherein $R^1$ is a lower alkyl group.
6. A compound of Claim 5, wherein $R^1$ is a methyl, ethyl or propyl group.
7. 2-(1-Decanoyloxypropyl) O-acetylsalicylate.
8. 2-(1-Decanoyloxypropyl) O-acetylsalicylate.
9. A process for preparing a compound as defined in Claim 1, comprising the step of reacting an acetyl salicylic acid derivative with a monoglycol ester having the formula :

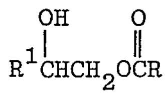

(III)

wherein R and $R^1$ are hydrocarbon radicals.

10. An analgesic and anti-inflammatory composition, comprising a compound of any one of Claims 1-8 in association with a pharmaceutically acceptable diluent or carrier therefor.

**Claims** (for the Contracting State AT)

1. A method for the preparation of an analgesic and anti-inflammatory composition, comprising associating with a pharmaceutically acceptable diluent or carrier a compound having the formula :

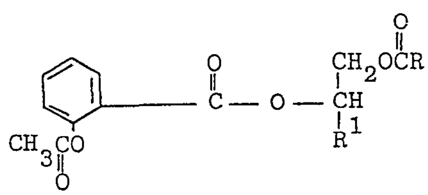

(I)

wherein R and $R^1$ are the same or different hydrocarbon radicals, or a pharmaceutically acceptable acid addition salt thereof.

2. A method of Claim 1, wherein R is an alkyl group having 1 to 15 carbon atoms.
3. A method of Claim 2, wherein said alkyl group is a straight chain alkyl group.
4. A method of Claim 3, wherein said straight chain alkyl group has 7 to 11 carbon atoms.
5. A method of any preceding claim, wherein $R^1$ is a lower alkyl group.
6. A method of Claim 5, wherein $R^1$ is a methyl, ethyl or propyl group.
7. A method of Claim 1, wherein said compound is 2-(1-decanoyloxypropyl) O-acetylsalicylate or 2-(1-dodecanoyloxypropyl) O-acetylsalicylate.
8. A process for preparing a compound of formula I as defined in Claim 1, comprising the step of reacting an acetyl salicylic acid derivative with a monoglycol ester having the formula :

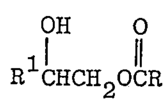

(III)

wherein R and $R^1$ are hydrocarbon radicals.

**Patentansprüche** (für die Vertragsstaaten : BE, CH, DE, FR, GB, IT, LI, NL, SE)

1. Verbindung mit der Formel

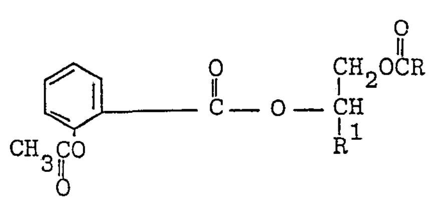

(I)

in welcher R und $R^1$ gleiche oder verschiedene Kohlenwasserstoffradikale darstellen oder ein pharmazeutisch akzeptierbares Säurezusatzsalz hiervon.

2. Verbindung gemäß Anspruch 1, in welcher R eine Alkylgruppe mit 1 bis 15 Kohlenstoffatomen ist.

3. Verbindung gemäß Anspruch 2, in welcher die Alkylgruppe eine geradkettige Alkylgruppe ist.

4. Verbindung gemäß Anspruch 3, in welcher die geradkettige Alkylgruppe 7 bis 11 Kohlenstoffatome aufweist.

5. Verbindung gemäß jedem der voraufgehenden Ansprüche, in welcher $R^1$ eine niedere Alkylgruppe ist.

6. Verbindung gemäß Anspruch 5, in welcher $R^1$ eine Methyl-, Äthyl- oder Propylgruppe ist.

7. 2-(1-Decanoyloxypropyl) O-Azetylsalizylat.

8. 2-(1-Dodecanoyloxypropyl) O-Azetylsalizylat.

9. Verfahren zur Herstellung einer Verbindung gemäß Anspruch 1 mit der Stufe, in der ein Azetylsalizylsäurederivat mit einem Monoglykolesther der Formel

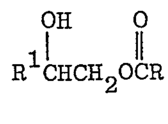

(III)

umgesetzt wird, wobei R und $R^1$ Kohlenwasserstoffradikale sind.

10. Schmerzlindernde und entzündungshemmende Zusammensetzung mit einer Verbindung gemäß einem der vorstehenden Ansprüche 1-8 zusammen mit einem dafür pharmazeutisch akzeptierbaren Lösungsmittel oder einer Trägersubstanz.

**Patentansprüche** (für den Vertragsstaat AT)

1. Verfahren zur Herstellung einer schmerzlindernden, entzündungshemmenden Zusammensetzung, bei dem eine Verbindung der Formel

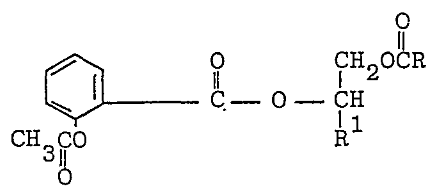

(I)

in der R und $R^1$ gleiche oder verschiedene Kohlenwasserstoffreste darstellen, oder ein pharmazeutisch akzeptierbares Säureadditionssalz hiervon, mit einem pharmazeutisch akzeptierbaren Verdünnungsmittel oder einer Trägersubstanz kombiniert wird.

2. Verfahren gemäß Anspruch 1, wobei R eine Alkylgruppe mit 1 bis 15 Kohlenstoffatomen ist.

3. Verfahren gemäß Anspruch 2, wobei die Alkylgruppe eine geradkettige Alkylgruppe ist.

4. Verfahren gemäß Anspruch 3, wobei die geradkettige Alkylgruppe 7 bis 11 Kohlenstoffatome aufweist.

5. Verfahren gemäß einem der voraufgehenden Ansprüche, wobei $R^1$ eine niedere Alkylgruppe ist.

6. Verfahren gemäß Anspruch 5, wobei $R^1$ eine Methyl-, Äthyl- oder Propylgruppe ist.

7. Verfahren gemäß Anspruch 1, wobei die Verbindung aus 2-(1-Decanoyloxypropyl) O-Acetylsalicylat oder 2-(1-Dodecanoyloxypropyl) O-Acetylsalicylat besteht.

8. Verfahren zur Herstellung der Verbindung gemäß Formel I aus Anspruch 1, mit einer Stufe, in der ein Acetylsalicylsäurederivat mit einem Monoglykolester der Formel

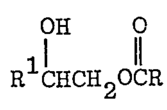

(III)

in der R und $R^1$ Kohlenwasserstoffreste sind, umgesetzt wird.

**Revendications** (pour les Etats contractants : BE, CH, DE, FR, GB, IT, LI, NL, SE)

1. Composé répondant à la formule :

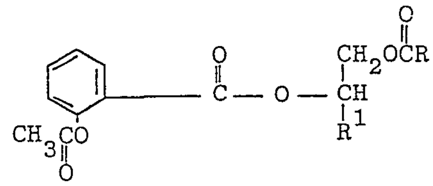

(I)

(dans laquelle R et $R^1$ sont des radicaux hydrocarbonés identiques ou différents) ou son sel d'addition d'acide pharmaceutiquement acceptable.

2. Composé selon la revendication 1, dans lequel R représente un groupe alkyle ayant 1 à 15 atomes de carbone.

3. Composé selon la revendication 2, dans lequel ledit groupe alkyle est un groupe alkyle linéaire.

4. Composé selon la revendication 3, dans lequel ledit groupe alkyle linéaire comporte 7 à 11 atomes de carbone.

5. Composé selon l'une quelconque des revendications précédentes, dans lequel $R^1$ est un groupe alkyle inférieur.

6. Composé selon la revendication 5, dans lequel $R^1$ est un groupe méthyle, éthyle ou propyle.

7. O-acétylsalicylate de 2-(1-décanoyloxypropyle).

8. O-acétylsalicylate de 2-(1-dodécanoyloxypropyle).

9. Procédé pour préparer un composé tel que défini à la revendication 1, comprenant l'étape consistant à faire réagir un dérivé de l'acide acétyl salicylique avec un ester de monoglycol répondant à la formule :

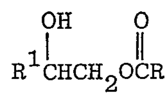

(III)

dans laquelle R et $R^1$ sont des radicaux hydrocarbonés.

10. Composé analgésique et anti-inflammatoire, comprenant un composé selon l'une quelconque des revendications 1 à 8 en association avec un diluant ou excipient, pharmaceutiquement acceptable, de ce composé.

**Revendications** (pour l'Etat contractant AT)

1. Procédé pour la préparation d'une composition analgésique et anti-inflammatoire, comprenant l'association, avec un diluant ou véhicule ou excipient pharmaceutiquement acceptable, d'un composé de formule :

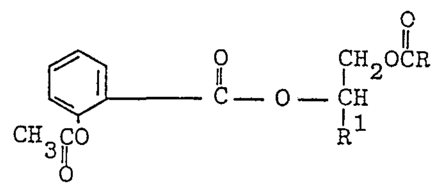

(I)

(dans laquelle R et $R^1$ sont des radicaux hydrocarbonés identiques ou différents), ou de son sel d'addition d'acide pharmaceutiquement acceptable.

2. Procédé selon la revendication 1, dans lequel R représente un groupe alkyle ayant 1 à 15 atomes de carbone.

3. Procédé selon la revendication 2, dans lequel ledit groupe alkyle est un groupe alkyle linéaire.

4. Procédé selon la revendication 3, dans lequel ledit groupe alkyle linéaire comporte 7 à 11 atomes de carbone.

5. Procédé selon l'une quelconque des revendications précédentes, dans lequel $R^1$ est un groupe alkyle inférieur.

6. Procédé selon la revendication 5, dans lequel $R^1$ est un groupe méthyle, éthyle ou propyle.

7. Procédé selon la revendication 1, dans lequel ledit composé est l'O-acétylsalicylate de 2-(1-décanoyloxypropyle) ou l'O-acétylsalicylate de 2-(1-dodécanoyloxypropyle).

8. Procédé pour préparer un composé de formule I, selon la définition donnée à la revendication 1, comprenant l'étape consistant à faire réagir un dérivé de l'acide acétyl salicylique avec un ester de monoglycol ayant la formule :

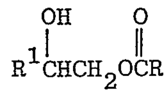 (III)

dans laquelle R et $R^1$ sont des radicaux hydrocarbonés.